Europäisches Patentamt

European Patent Office

Office européen des brevets

(11) Publication number: **0 049 851**

**B1**

(12) **EUROPEAN PATENT SPECIFICATION**

(45) Date of publication of patent specification: **09.01.85**

(51) Int. Cl.⁴: **A 61 B 1/00, G 02 B 23/00**

(21) Application number: **81107965.6**

(22) Date of filing: **06.10.81**

(54) Endoscope.

(30) Priority: **09.10.80 JP 141757/80**

(43) Date of publication of application:
**21.04.82 Bulletin 82/16**

(45) Publication of the grant of the patent:
**09.01.85 Bulletin 85/02**

(84) Designated Contracting States:
**AT BE CH DE FR GB IT LI NL SE**

(56) References cited:
**DE-A-2 653 661
DE-A-2 853 466
GB-A- 993 325
US-A-3 915 157**

(73) Proprietor: **OLYMPUS OPTICAL CO., LTD.**
**43-2, 2-chome, Hatagaya Shibuya-ku**
**Tokyo 151 (JP)**

(72) Inventor: **Ueba, Yasuhiro**
**1-22-15, Tokura**
**Kokubunji-shi Tokyo (JP)**

(74) Representative: **Freischem, Werner, Dipl.-Ing.**
**et al**
**Patentanwälte Dipl.-Ing. W. Freischem Dipl.-Ing.**
**I. Freischem An Gross St. Martin 2**
**D-5000 Köln 1 (DE)**

Courier Press, Leamington Spa, England.

## Description

This invention relates to an endoscope with an instrument raising unit.

In general, endoscopes are provided with an insertion channel in which a medical instrument such as a forceps is inserted and guided, and a raising block for restricting the direction in which the instrument is led is disposed at the distal opening portion of the insertion channel. The raising block can be remotely rocked by a proximal control section through means of a control wire passed through the endoscope. Since the distal end portion of the instrument, which is urged curvedly to project, abuts strongly on the raising block, there is caused a great resistance, making smooth operation difficult.

In order to eliminate such difficulty, there has conventionally been proposed a system in which a pair of operating pins are attached to a rocking body (Japanese Patent Publication No. 17679/77).

In this system, however, a medical instrument to be bent is caused to abut on the pair of operating pins which are rocking together with the rocking body, so that the distance between the points of contact between the instrument and the operating pins is fixed. Therefore, the force to rock the rocking body is too great to ensure smooth operation.

In the aforesaid conventional system, moreover, both of the operating pins strongly press on the instrument as they slide on the instrument, and the contact points of these pins on the instrument move along the longitudinal direction of the instrument. Accordingly, the length of projection of the instrument in the body cavity greatly changes during the raising operation to constitute a hindrance to the operation. In consideration of the narrowness of the body cavity, moreover, such change is quite hazardous.

US—A—3 915 157 discloses an endoscope including all the features of the prior art portion of claim 1. In particular there are disclosed instrument raising means including a rocking body inside a chamber and capable of rocking around an axis at right angles to the extending direction of the medical instrument and an operating member in the rocking body and rotatable therewith.

DE—A—2 953 466 discloses a variety of instrument raising means comprising a rocking body including operating means rockable therewith and a cooperating fixed member which, when the body is rocked by the action of driving means, bend the instrument about the fixed member to raise (or lower) it by a lever action.

In the prior art, the distance between the operating point and the fulcrum, i.e., the length of the bent portion of the medical instrument, e.g. forceps, is diminished as the bending angle of the said instrument is increased. In the endoscope according to US—A—3 915 157 the distance between the fulcrum and the operating point remains substantially constant regardless of the bending angle of the medical instrument. The rotating angle of the rocking body is increased if the bending angle of the forceps is increased, with the result that the operating point gets somewhat closer to the fulcrum. In the endoscope according to DE—A—2 853 466 the operating point gets closer to the fulcrum (i.e., the length of the bent portion is decreased), if the bending angle of the forceps is made greater.

The object of this invention is to provide an endoscope capable of safe and smooth raising of a medical instrument without involving any great change in the length of projection of the instrument from the endoscope.

This invention can be more fully understood from the following detailed description when taken on conjunction with the accompanying drawings, in which:

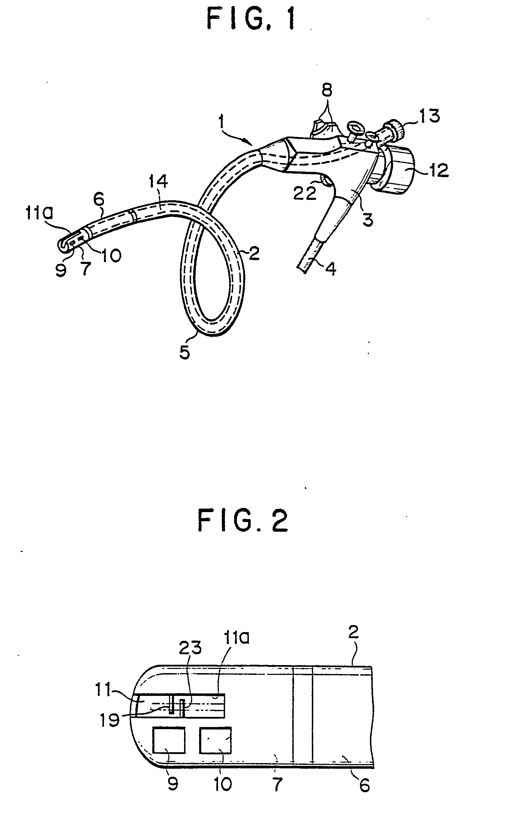

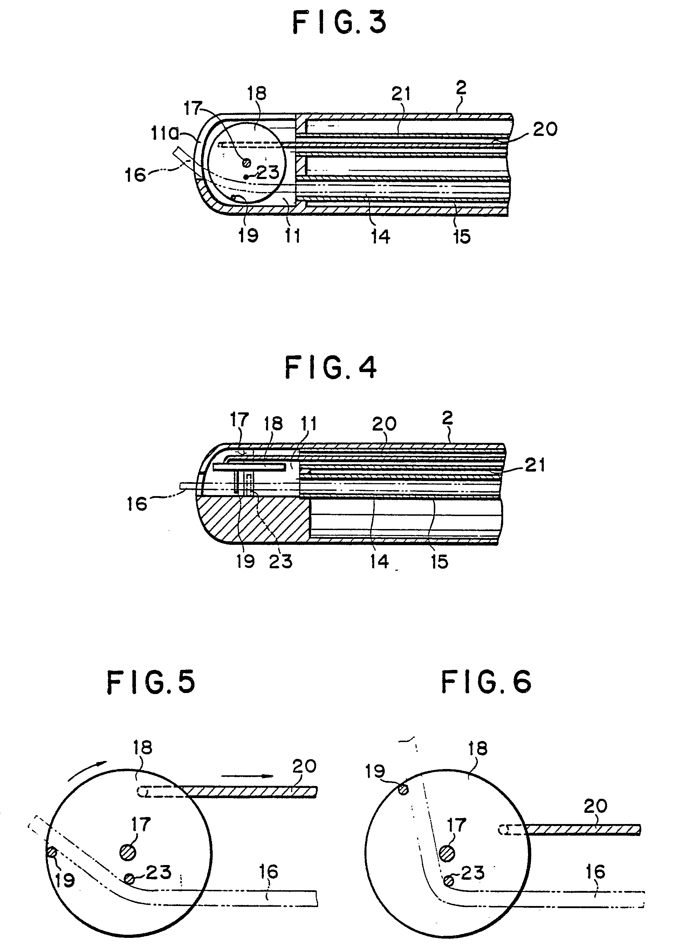

Figs. 1 to 6 show an endoscope according to an embodiment of this invention, in which Fig. 1 is an overall schematic perspective view, Fig. 2 is a plan view of a distal rigid portion, Figs. 3 and 4 are sectional views of the rigid portion shifted at an angle of 90°, and Figs. 5 and 6 are side views of a rocking body in different rotating positions showing the raised state of a medical instrument.

Now there will be described an embodiment of this invention with reference to the accompanying drawings.

In Fig. 1, numeral 1 designates an endoscope body which includes a flexible insertion section 2 and a control section 3 connected with the proximal end of the insertion section 2. A light guide cable 4 is coupled to the control section 3. The insertion section 2 is formed of a flexible tube 5 and a distal rigid portion 7 coupled to the distal end of the flexible tube 5 by means of a bendable tube 6 connected therebetween. The bendable tube 6 can be remotely curved by means of a pair of angle knobs 8 at the control section 3. As shown in Fig. 2, moreover, an illumination window 9 and an observation window 10 are arranged on the top side of the distal rigid portion 7 along the longitudinal direction of the insertion section 2. Further, an opening portion 11a of an instrument raising chamber 11 is formed in parallel with the windows 9 and 10. The illumination window 9 is connected with a light guide (not shown) which is passed through the insertion section 2, the control section 3, and the light guide cable 4, while the observation window 10 is connected with an eyepiece section 12 at the control section 3 by means of an image guide (not shown) which is passed through the insertion section 2 and the control section 3.

Formed at the insertion section 2 and the control section 3 of the endoscope body 1, as shown in Figs. 3 and 4, is a channel 14 for instrument insertion whose distal end opens into the raising chamber 11 and whose proximal end is connected with an insertion port 13 at the control section 3. The channel 14 is formed of the bore of a flexible tube 15.

Disposed in the instrument raising chamber 11 is a discoid rocking body or rotating disc 18 with a rotating center shaft 17 extending in a direction at right angles to the direction in which a medical instrument 16 such as a forceps is inserted. Each end of the rotating shaft 17 is rockably supported on its corresponding side wall of the rigid portion 7. As shown in Fig. 4, the rocking body 18 is located in a position kept aside from the passage of the instrument 16, and an operating member or pin 19 protrudes from one side of the rocking body 18 near the periphery thereof toward the passage of the instrument 16. The tip end of a control wire 20 is fixed on the other side of the rocking body 18 near the upper peripheral portion thereof. The other end of the control wire 20 is led to the control section 3 through a flexible guide tube 21 which is passed through the insertion section 2 and the control section 3, and is coupled to a raising knob 22 rockably supported by the control section 3. By rocking the knob 22 in one direction, the control wire 20 can be pulled so that the rocking body 18 may be rocked in the clockwise direction of Fig. 3.

Inside the instrument raising chamber 11, a fixed member or pin 23 protrudes toward the passage of the instrument 16 from the wall portion of the chamber 11 lying across the passage of the instrument 16 from the aforesaid one side of the rocking body 18. Projecting at right angles to the direction of insertion of the instrument 16, the fixed pin 23 is deviated downwardly from the rotating shaft 17 of the rocking body 18. As shown in Fig. 3, the fixed pin 23 is nearer to the rotating shaft 17 than the operating pin 19 is.

Further, both the operating pin 19 and the fixed pin 23 project into the passage of the instrument 16 inside the instrument raising chamber 11 so that their respective peripheral side faces may abut on the instrument 16. In a normal stand-by state, the operating pin 19 and the fixed pin 23 are located in the positions shown in Fig. 3, that is, the operating pin 19 is located in a lower position so that these two pins 19 and 23 and the rotating shaft 17 are substantially in a straight line, allowing the instrument 16 projecting from the channel 14 to advance between the pins 19 and 23.

Now there will be described the raising operation in the aforementioned endoscope.

In the normal state, the control wire 20 is advanced, and the rocking body 18 is in the position shown in Fig. 3. When the instrument 16 is inserted, the tip end portion of the instrument 16 passes between the operating pin 19 and the fixed pin 23 to project out of the rigid portion through the opening portion of the instrument raising chamber 11. At this time, the instrument 16 makes a substantially straight advance as indicated by a chain line.

Then, in raising the instrument 16, the knob 22 at the control section 3 is rocked in one direction to pull the control wire 20, thereby rocking the rocking body 18 clockwise, as shown in Fig. 5. Accompanying the rocking motion of the rocking body 18, the operating pin 19 (point of action) raises the forward end side of the instrument 16 while the fixed pin 23 (fulcrum) sustains the rear end side portion of the instrument 16, so that the forward end side of the instrument 16 can rise. While the instrument 16 is supported at a fixed point by the fixed pin 23, the point of the instrument 16 abutting on the operating pin 19 moves forward or away from the fixed point as the rocking body 18 rocks. As shown in Figs. 3, 5 and 6, therefore, the distance between the operating pin 19 and the fixed pin 23 continuously increases with the rocking motion of the rocking body 18. In other words, the distance between the fulcrum and the point of action increases with the rise. Thus, the raising operation requires only small force. Since the point (fulcrum) in contact with the fixed pin 23 is fixed, moreover, the raising operation is stable.

Although a disc is used for the rocking body in the above embodiment, this invention is not limited to such configuration. Further, the pins used as the operating and fixed members may be replaced with members in any other styles, provided that they project toward the instrument so as to be able to abut on the instrument being raised. For example, a cam projection formed integrally with the rocking body may be used for the operating member. The means for rocking the rocking body is not limited to the wire, and may be any suitable means which can rock the rocking body in any direction.

According to this invention, as described above, the distance between the point of action provided by the operating member and the fulcrum provided by the fixed member increases with the rise of the instrument, so that the instrument can be raised with a small operating force, ensuring smooth and easy operation. Moreover, the use of the fixed member as the fulcrum enables smooth and stable raising of the instrument, and prohibits the length of projection of the instrument from changing. Thus, the raising operation in the narrow body cavity can safely be performed without hindrance.

**Claims**

1. An endoscope with an elongate medical instrument (16), comprising: an elongated insertion section (2) to be inserted in the human body; and a control section (3) coupled to the proximal end of said insertion section (2), said insertion section (2) including a chamber (11) formed in the vicinity of the distal end (7) of said insertion section (2), said chamber (11) having an opening (11a) through which a tip end portion of said instrument (16) is led out of said endoscope, a channel (14) having one and the other ends thereof connected with said chamber (11) and said control section (3) respectively and extending through and along said insertion section (2), instrument raising means provided in the chamber (11) for raising the protruding tip of the medical instrument and means (20) for driving the instrument raising means, said instrument raising means include a rocking body (18), capable of rocking around an axis (17) at right angles to the extending direction of said instrument (16) and, spaced from said axis (17), an operating member (19) located on the rocking body (18) and rotatable therewith, characterized in that a fixed member (23) is located in said chamber (11) nearer to the axis (17) of said rocking body (18) than said operating member (19), so as to face said operating member (19) and spaced therefrom so that in an initial position of the rocking body (18) the connecting line between the axis (17) and the operating member (23) crosses the extending direction of said instrument (16), said instrument extends between said operating member (19) and said fixed member (23), whereby said instrument (16) is caused to abut on said fixed member (23) and said operating member (19) by said rocking body (18) rotating in one direction and is bent about said fixed member (23) by said operating member (19), while the distance between the operating member (19) and the fixed member (23) increases with the rotation in the one direction of the rocking body (18).

2. An endoscope according to claim 1 wherein said rocking body (18) is a disc, said operating member (19) protruding from one side of said disc.

3. An endoscope according to claim 2, wherein said operating member (19) is an operating pin protruding from the peripheral portion of said disc, and said fixed member (23) is a fixed pin disposed near said axis (17), said two pins (19, 23) extending substantially in parallel with each other.

**Patentansprüche**

1. Endoskop mit einem länglichen medizinischen Instrument (16), bestehend aus einem der Einführung in den menschlichen Körper dienenden länglichen Einführungsteil (2) und einem am hinteren Ende desselben angebrachten Manipulationsteil (3), wobei der Einführungsteil (2) mit einer am vorderen Ende (7) ausgebildeten Kammer (11) versehen ist, welche eine Öffnung (11a) aufweist, durch die ein Vorderteil des Instruments (16) aus dem Endoskop hinaus geführt ist, und weiter aus einem Kanal (14), der längs durch den Einführungsteil (2) hindurch geht und mit seinen beiden Enden an die Kammer (11) bzw. den Manipulationsteil (3) angeschlossen ist, wobei zum Anheben des herausragenden Vorderteils des medizinischen Instruments in der Kammer (11) ein Instrumentenanheber und eine Antriebseinrichtung (20) zum Antrieb des Instrumentenanhebers vorgesehen sind, der seinerseits aus einem um eine zur Längsrichtung des Instruments (16) rechtwinklige Achse (17) drehbaren Schwenkstück (18) und einem darauf zusammen mit ihm drehbaren und mit Abstand von der Achse (17) sitzenden Stellglied (19) besteht, dadurch gekennzeichnet, daß in der Kammer (11) ein näher als das Stellglied (19) bei der Achse (17) des Schwenkstücks (18) gelegenes festes Abstützglied (23) vorgesehen ist, so daß es derart im Abstand vom Stellglied (19) steht, daß die Verbindungslinie zwischen Achse (17) und Stellglied (23) in der Ausgangsstellung des Schwenkstücks (18) die Längsrichtung des Instruments (16) kreuzt, wobei dieses zwischen dem Stellglied (19) und dem festen Abstützglied (23) hindurch geht, so daß sich das Instrument (16) unter der Wirkung des in einer Richtung gedrehten Schwenkstücks (18) an das feste Abstützglied (23) und das Stellglied (19) anlegt und durch das Stellglied (19) um das feste Abstützglied (23) herum gebogen wird, während der Abstand zwischen dem Stellglied (19) und dem festen Abstützglied (23) mit der Drehung des Schwenkstücks (18) in dieser einen Richtung wächst.

2. Endoskop nach Anspruch 1, dadurch gekennzeichnet, daß das Schwenkstück (18) eine Scheibe ist, und daß das Stellglied (19) von einer Seite dieser Scheibe absteht.

3. Endoskop nach Anspruch 2, dadurch gekennzeichnet, daß das Stellglied (19) ein vom Rand der Scheibe abstehender Stellstift ist, während das feste Abstützglied (23) eine nahe der Achse (17) angeordneter feststehender Stift ist, wobei beide Stifte (19, 23) parallel zueinander verlaufen.

**Revendications**

1. Endoscope avec un instrument médical allongé (16) comprenant: une sonde allongée (2) destinée à être introduite dans le corps humain et un boîtier de commande (3) accouplé à l'extrémité proximale de ladite sonde (2), ladite sonde (2) comprenant une chambre (11) disposée à proximité de l'extrémité distale (7) de ladite sonde (2), ladite chambre (11) comportant une lumière (11a) à travers laquelle la partie terminale portant la pointe dudit instrument (16) est sortie hors dudit endoscope, un canal (14) dont l'une des extrémités est raccordée à ladite chambre (11) et l'autre extrémité audit boîtier de commande (3), ledit canal cheminant à travers et le long de ladite sonde (2), un dispositif d'érection d'instrument aménagé dans la chambre (11) pour lever la pointe protubérante de l'instrument médical et un mécanisme (20) pour assurer l'entraînement du dispositif d'érection, ledit dispositif d'érection comprenant un basculeur (18) susceptible de basculer autour d'un axe (17) perpendiculaire à la direction du déploiement dudit instrument (16) et un organe de manoeuvre (19) disposé à l'écart dudit axe (17) et fixé audit basculeur (18) dont il est solidaire de la rotation, caractérisé par le fait qu'un organe fixe (23) est logé dans ladite chambre (11) plus proche de l'axe (17) dudit basculeur (18) que ledit organe de manoeuvre (19) de façon à être en regard dudit organe de manoeuvre (19) et disposé à l'écart dudit dernier organe en sorte que, dans la position d'origine du basculeur (18), la droite reliant l'axe (17) et l'organe de manoeuvre (19) coupe la ligne de déploiement dudit instrument (16), étant entendu que ledit instrument chemine entre ledit organe de manoeuvre (19) et ledit organe fixe (23), disposition par laquelle ledit instrument (16) est appuyé contre ledit organe fixe (23) et contre ledit organe de manoeuvre (19) sous l'action du basculeur (18) tournant dans un sens et est cintré autour dudit organe fixe (23) par ledit organe de manoeuvre (19), tandis que l'écartement entre l'organe de manoeuvre (19) et l'organe fixe (23) augmente en fonction de la rotation dans un sens du basculeur (18).

2. Endoscope suivant la revendication 1, caractérisé par le fait que ledit basculeur (18) est constitué par un disque, ledit organe de manoeuvre (19) formant une protubérance sur une des faces dudit disque.

3. Endoscope suivant la revendication 2, caractérisé par le fait que ledit organe de manoeuvre (19) est constitué par un ergot de commande formant une protubérance sur la partie périphérique dudit disque et ledit organe fixe (23) est un ergot fixe disposé à proximité dudit axe (17), lesdits deux ergots (19, 23) se déployant de façon sensiblement parallèle.

# FIG. 1

# FIG. 2

0 049 851

# FIG.3

# FIG.4

# FIG.5

# FIG.6

2